# EUROPÄISCHE PATENTSCHRIFT

(11) **EP 3 834 717 B1**
(45) Veröffentlichungstag und Bekanntmachung des Hinweises auf die Patenterteilung: **18.09.2024**
(21) Anmeldenummer: 19215363.3
(22) Anmeldetag: 11.12.2019
(51) Int. Cl.: A61B 5/055, A61B 6/03, A61B 6/04

(54) **VERFAHREN ZU EINEM POSITIONIEREN EINES PATIENTEN**
METHOD FOR POSITIONING A PATIENT
PROCÉDÉ DE POSITIONNEMENT D'UN PATIENT

(43) Veröffentlichungstag der Anmeldung: 16.06.2021
(73) Patentinhaber: Siemens Healthineers AG, 91301 Forchheim (DE)
(72) Erfinder: Beck, Thomas, 91052 Erlangen (DE)
(74) Vertreter: Siemens Healthineers Patent Attorneys

(56) Entgegenhaltungen:
- WO-A1-2019/216896
- DE-U1- 202014 008 084
- US-A1- 2008 194 942
- US-A1- 2013 148 778

## Beschreibung

Die vorliegende Erfindung betrifft ein Verfahren zu einem Positionieren eines Patienten für eine medizinische Bildgebungsuntersuchung nach Anspruch 1.

Für die Durchführung von medizinischen Bildgebungsuntersuchungen, wie beispielsweise Magnetresonanzuntersuchungen, an einem Patienten ist während einer Vorbereitung der medizinischen Bildgebungsuntersuchung eine Positionierung eines Patienten, insbesondere eines zu untersuchenden Bereichs des Patienten, in einem Isozentrum erforderlich. Bei der Positionierung des Patienten wird bevorzugt der zu untersuchende Bereich des Patienten in einem Isozentrum einer medizinischen Bildgebungsvorrichtung, beispielswiese einer Magnetresonanzvorrichtung, angeordnet und/oder positioniert.

Eine derartige Positionierung kann beispielsweise mittels eines Positionierungslasers erfolgen, der an einer Eingangsöffnung eines Patientenaufnahmebereichs der medizinischen Bildgebungsvorrichtung angeordnet ist. Mittels eines derartigen Positionierungslasers, der ein Markierungselement, beispielsweise ein Markierungskreuz, auf den Patienten projiziert, wird zunächst der zu untersuchende Bereich des Patienten markiert. Da ein Abstand von dem Positionierungslaser zu einem Isozentrum der medizinischen Bildgebungsvorrichtung bekannt ist, wird anschließend der Patiententisch derart um den Abstand verschoben. Jedoch bedeutet die Verwendung eines Positionierungslasers auch immer eine gewisse Gefahr für den Patienten. Beispielsweise ist bei Kopfuntersuchungen darauf zu achten, dass der Positionierungslaser nicht auf die Augen des Patienten projiziert.

Eine weitere Möglichkeit, die Position des zu untersuchenden Bereichs des Patienten für eine medizinische Bildgebungsuntersuchung festzulegen, ist die Verwendung einer Positionsmarkierungseinheit direkt an der Patientenlagerungsvorrichtung. Bei einer derartigen Positionsbestimmung erfolgt eine Kontrolle der Position mittels einer Ausgabeeinheit, insbesondere mittels eines Displays, das an einem Scanner der medizinischen Bildgebungsvorrichtung angeordnet ist. Jedoch befindet sich häufig der Benutzer zu weit entfernt von dem Display, um die Position zu kontrollieren. Zudem ist die Darstellung auf dem Display nur sehr grob, so dass eine Fehlpositionierung von bis zu 30 cm für den Benutzer kaum ersichtlich ist.

Aus US 2008 0 194 942 A1 ist eine Patientenlagerungsvorrichtung bekannt, die eine Positionseingabeeinheit an einem seitlichen Randbereich eines Patiententischs umfasst.

DE 20 2014 008 084 U1 offenbart ein medizinisches Gerät, das wenigstens eine streifenförmige, berührungsempfindliche Eingabevorrichtung umfasst.

WO 2019 216 896 A1 beschreibt ein medizinisches Patientenbett mit einem visuellen Anzeigesystem. Das visuelle Anzeigesystem umfasst einen oder mehrere Lichtstreifen, die am Patientenbett angeordnet sind. Mittels des visuellen Anzeigesystems kann eine zu untersuchende Position und/oder Bereich angezeigt werden.

US 2013 0 148 778 A1 offenbart eine Patientenlagerungsvorrichtung für ein Bildgebungssystem. Die Patientenlagerungsvorrichtung umfasst eine Scanpositionskennung, die ein Signal erzeugt, das auf mindestens eine Startscanposition oder eine Endscanposition für eine vorbestimmte Region eines Subjekts, insbesondere eines Patienten, hinweist.

Der vorliegenden Erfindung liegt insbesondere die Aufgabe zugrunde, eine einfache Positionierung eines Patienten in einem Patientenaufnahmebereich mit einer direkten Kontrolle der Positionierung für einen Benutzer bereitzustellen. Die Aufgabe wird durch die Merkmale des unabhängigen Anspruchs 1 gelöst.

Vorteilhafte Ausgestaltungen sind in den Unteransprüchen beschrieben.

Die Patientenlagerungsvorrichtung umfasst bevorzugt eine Basiseinheit, wobei der Patiententisch der Patientenlagerungsvorrichtung bezüglich der Basiseinheit bewegbar ausgebildet ist. Der Patiententisch ist hierbei insbesondere in Richtung einer Längserstreckung und/oder einer Längsrichtung des Patiententischs bewegbar ausgebildet. Zudem kann der Patiententisch auch in vertikaler Richtung bewegbar ausgebildet sein, um ein Aufsitzen eines Patienten auf den Patiententisch zu erleichtern.

Der Patiententisch umfasst bevorzugt einen Lagerungsbereich zu einer Lagerung und/oder Positionierung des Patienten für eine medizinische Bildgebungsuntersuchung. Für eine medizinische Bildgebungsuntersuchung wird der Patient bevorzugt auf dem Patiententisch, insbesondere auf dem Lagerungsbereich des Patiententischs, liegend in den Patientenaufnahmebereich der medizinischen Bildgebungsvorrichtung eingefahren. Der Patiententisch umfasst zwei seitliche Randbereiche. Zwischen einem linken seitlichen Randbereich und einem rechten seitlichen Randbereich ist die Lagerungsfläche des Patiententischs angeordnet. Insbesondere ist die Lagerungsfläche in Quererstreckung des Patiententischs zwischen den beiden seitlichen Randbereichen angeordnet. Die Quererstreckung ist bevorzugt quer und/oder senkrecht zur Längserstreckung des Patiententischs ausgebildet.

Die medizinische Bildgebungsvorrichtung kann beispielsweise eine Computertomographie-Vorrichtung und/oder eine Positron-Emissions-Tomographie-Vorrichtung (PET-Vorrichtung) umfassen. Besonders vorteilhaft umfasst die medizinische Bildgebungsvorrichtung eine Magnetresonanzvorrichtung.

Die medizinische Bildgebungsvorrichtung umfasst bevorzugt eine Scannereinheit, die zumindest teilweise den Patientenaufnahmebereich umgibt. Der Patientenaufnahmebereich ist zu einer Aufnahme des Patienten, insbesondere des zu untersuchenden Bereichs des Patienten, für eine medizinische Bildgebungsuntersuchung ausgelegt und/oder ausgebildet. Beispielsweise ist hierzu der Patientenaufnahmebereich zylinderförmig ausgebildet und/oder zylinderförmig von der Scannereinheit umgeben. Innerhalb des Patientenaufnahmebereich ist bevorzugt ein Field of View (FOV) und/oder ein Isozentrum der medizinischen Bildgebungsvorrichtung angeordnet. Das FOV umfasst bevorzugt einen Erfassungsbereichs der medizinischen Bildgebungsvorrichtung, innerhalb dessen die Bedingungen für eine Erfassung von medizinische Bilddaten innerhalb des Patientenaufnahmebereichs vorliegen. Das Isozentrum der medizinische Bildgebungsvorrichtung umfasst bevorzugt den Bereich und/oder Punkt innerhalb der medizinischen Bildgebungsvorrichtung, der die optimalsten und/oder idealsten Bedingungen für die Erfassung von medizinischen Bilddaten aufweist. Beispielsweise umfasst das Isozentrum den homogensten Magnetfeldbereich innerhalb einer als Magnetresonanzvorrichtung ausgebildeten medizinischen Bildgebungsvorrichtung.

Der Patiententisch ist insbesondere derart ausgebildet, dass ein kollisionsfreies Einfahren des Patiententischs in den Patientenaufnahmebereich der medizinischen Bildgebungsvorrichtung möglich ist. Zudem ist die Patientenlagerungsvorrichtung bei einer als Magnetresonanzvorrichtung ausgebildeten medizinischen Bildgebungsvorrichtung magnetresonanzkompatibel ausgebildet.

Die Positionierungseinheit der Patientenlagerungsvorrichtung ist zu einer Positionierung des Patiententischs ausgebildet. Hierzu weist die Positionierungseinheit die Positionseingabeeinheit und die Positionsausgabeeinheit auf. Zudem kann die Positionierungseinheit auch eine Antriebseinheit zur Generierung einer Antriebsenergie und/oder eines Antriebsmoments zur Bewegung und/oder Positionierung des Patiententischs aufweisen. Des Weiteren kann die Positionierungseinheit auch eine Recheneinheit umfassen zur Steuerung der Bewegung und/oder Positionierung des Patiententischs. Alternativ oder zusätzlich kann die Steuerung des Patiententisch auch mittels einer Steuerungseinheit und/oder Recheneinheit der medizinischen Bildgebungsvorrichtung erfolgen.

Die Positionseingabeeinheit umfasst bevorzugt zumindest ein Positionseingabeelement. Das zumindest eine Positionseingabeelement ist hierbei an dem seitlichen Randbereich des Patiententischs angeordnet. Besonders vorteilhaft ist die Positionseingabeeinheit, insbesondere das zumindest eine Positionseingabeelement, direkt neben dem Lagerungsbereich an dem Patiententisch angeordnet.

Mittels der Positionseingabeeinheit kann von einem Benutzer, insbesondere einem medizinischen Bedienpersonal, eine Position, insbesondere eine Untersuchungsposition, eingegeben werden. Die eingegebene Position, insbesondere die eingegebene Untersuchungsposition, umfasst dabei diejenige Position des Patienten und/oder Patiententischs, die während der medizinischen Bildgebungsuntersuchung innerhalb des FOVs und/oder des Isozentrums der medizinischen Bildgebungsvorrichtung positioniert sein soll. Von einem Benutzer, beispielsweise einem medizinischen Bedienpersonal, kann hierbei der zu untersuchende Bereich als Untersuchungsposition mittels der der Positionseingabeeinheit durch Eingabe der Position festgelegt werden. Besonders vorteilhaft ist der Patiententisch und/oder die Patientenlagerungsvorrichtung derart ausgebildet, dass nach Eingabe der Position, insbesondere der Untersuchungsposition, an der Positionseingabeeinheit durch den Benutzer der Patiententisch automatisch in diejenige Position fährt und/oder bewegt wird, dass die vom Benutzer eingegebene Position mit dem Isozentrum der medizinischen Bildgebungsvorrichtung übereinstimmt.

Die Positionsausgabeeinheit umfasst bevorzugt zumindest ein Positionsausgabeelement. Das zumindest eine Positionsausgabeelement ist hierbei an dem seitlichen Randbereich des Patiententischs angeordnet. Besonders vorteilhaft ist die Positionsausgabeeinheit, insbesondere das zumindest eine Positionsausgabeelement, direkt neben dem Lagerungsbereich an dem Patiententisch angeordnet.

Die Positionseingabeeinheit, insbesondere das zumindest eine Positionseingabeelement, und die Positionsausgabeeinheit, insbesondere das zumindest eine Positionsausgabeelement, können hierbei auch einstückig und/oder einteilig ausgebildet sein. Beispielsweise kann hierbei die Positionseingabeeinheit, insbesondere das zumindest eine Positionseingabeelement, und die Positionsausgabeeinheit, insbesondere das zumindest eine Positionsausgabeelement, als berührsensitive und/oder drucksensitive Leuchtelemente ausgebildet sein, die bei einer Berührung durch den Benutzer aufleuchten.

Durch die Ausgestaltung kann vorteilhaft eine direkte Eingabe der Untersuchungsposition für eine Positionierung des Patiententischs innerhalb eines Isozentrums der medizinischen Bildgebungsvorrichtung erfolgen, ohne dass vorher der Patiententisch von einem Benutzer in eine definierte Position bewegt werden muss. Zudem kann auch direkt ein Feedback und/oder ein Positionskontrollsignal an den Benutzer, insbesondere an ein medizinisches Bedienpersonal, mittels der Positionsausgabeeinheit ausgegeben werden. Somit erhält der Benutzer auch direkt oder unmittelbar nach der Eingabe der Untersuchungsposition eine Bestätigung für seine Eingabe. Insbesondere kann die Ausgabe eines Feedbacks und/oder eines Positionskontrollsignals an den Benutzer an einem Ort und/oder einer Position erfolgen, die in Richtung einer Längserstreckung des Patiententischs mit einer Position der Positionseingabe mittels der Positionseingabeeinheit übereinstimmt, so dass die Ausgabe direkt im Sichtfeld des Benutzers erfolgen kann. Besonders vorteilhaft kann hierdurch ein hoher Bedienkomfort für den Benutzer während einer Vorbereitung des Patienten bereitgestellt werden. Weiterhin können derart insbesondere Fehlpositionierungen des Patienten innerhalb des Patientenaufnahmebereichs reduziert und/oder verhindert werden. Des Weiteren kann derart auch eine Vorbereitungszeit zur Vorbereitung des Patienten für die medizinische Bildgebungsuntersuchung reduziert werden und damit auch ein hoher Patientendurchsatz ermöglicht werden.

In einer vorteilhaften Weiterbildung der Patientenlagerungsvorrichtung kann es vorgesehen sein, dass die Positionseingabeeinheit eine Länge umfasst, die im Wesentlichen einer Länge des Patiententischs entspricht. Bevorzugt entspricht die Länge der Positionseingabeeinheit hierbei im Wesentlichen einer Länge des Lagerungsbereichs des Patiententischs. Die Positionseingabeeinheit kann dabei ein Positionseingabeelement umfassen, wobei eine Länge des Positionseingabeelements im Wesentlichen einer Länge des Patiententischs entspricht. Umfasst die Positionseingabeeinheit mehrere Positionseingabeelemente, die in Richtung einer Längserstreckung des Patiententischs nacheinander angeordnet sind, so kann im Wesentlichen eine Länge der mehreren Positionseingabeelemente einer Länge des Patiententischs entsprechen. Hierbei soll unter einer Länge, die im Wesentlichen einer Länge des Patiententischs entspricht, insbesondere verstanden werden, dass die Länge der Positionseingabeeinheit mindestens 80 % der Länge des Patiententischs, insbesondere mindestens 80 % einer Länge des Lagerungsbereichs des Patiententischs, entspricht. Besonders vorteilhaft entspricht die Länge der Positionseingabeeinheit mindestens 85 % der Länge des Patiententischs, insbesondere mindestens 85 % der Länge des Lagerungsbereichs des Patiententischs. Besonders vorteilhaft entspricht die Länge der Positionseingabeeinheit mindestens 90 % der Länge des Patiententischs, insbesondere mindestens 90 % der Länge des Lagerungsbereichs des Patiententischs. Besonders vorteilhaft entspricht die Länge der Positionseingabeeinheit mindestens 95 % der Länge des Patiententischs, insbesondere mindestens 95 % der Länge des Lagerungsbereichs des Patiententischs. Besonders vorteilhaft entspricht die Länge der Positionseingabeeinheit mindestens 98 % der Länge des Patiententischs, insbesondere mindestens 98 % der Länge des Lagerungsbereichs des Patiententischs.

Hierdurch kann vorteilhaft eine Eingabe an der Positionseingabeeinheit von dem Benutzer, insbesondere einem medizinischen Bedienpersonal, direkt an der Position der Positionseingabeeinheit erfolgen, wobei die Position in Richtung einer Längserstreckung einer Position des zu untersuchenden Bereichs des Patienten entspricht. Insbesondere kann derart bereits bei einer Vorbereitung des zu untersuchenden Bereichs des Patienten die Untersuchungsposition an der Positionseingabeeinheit festgelegt werden, ohne dass hierbei der Benutzer seine Position zur Vorbereitung des Patienten verlassen muss.

In einer vorteilhaften Weiterbildung der Patientenlagerungsvorrichtung kann es vorgesehen sein, dass die Positionseingabeeinheit zumindest ein berührsensitives und/oder drucksensitives Positionseingabeelement umfasst. Das berührsensitive und/oder drucksensitive Positionseingabeelement ist bevorzugt dazu ausgebildet, eine Positionseingabe durch ein Betätigen, insbesondere ein Drücken, eines berührsensitiven und/oder drucksensitiven Bereichs des Positionseingabeelements zu Erfassen. Das Betätigen, insbesondere das Drücken, des berührsensitiven und/oder drucksensitiven Bereich des Positionseingabeelements erfolgt bevorzugt durch den Benutzer, insbesondere ein medizinisches Bedienpersonal. Hierdurch kann eine besonders einfache Eingabe einer Position, insbesondere einer Untersuchungsposition, für einen Benutzer erfolgen und damit eine hoher Bedienkomfort für eine Vorbereitung eines Patienten für eine medizinische Bildgebungsuntersuchung bereitgestellt werden. Insbesondere kann derart eine intuitive Eingabe für den Benutzer bereitgestellt werden.

In einer vorteilhaften Weiterbildung der Patientenlagerungsvorrichtung kann es vorgesehen sein, dass das berührsensitive und/oder drucksensitive Positionseingabeelement zwei oder mehr berührsensitive und/oder drucksensitive Bereiche umfasst, wobei die einzelnen berührsensitiven und/oder drucksensitiven Bereiche in Richtung einer Längserstreckung des berührsensitiven und/oder drucksensitiven Positionseingabeelements nacheinander angeordnet sind. Vorzugsweise weisen die einzelnen berührsensitiven und/oder drucksensitiven Bereiche eine maximale Länge von 15 cm auf. Besonders vorteilhaft weisen die einzelnen berührsensitiven und/oder drucksensitiven Bereiche eine maximale Länge von 10 cm auf. Besonders vorteilhaft weisen die einzelnen berührsensitiven und/oder drucksensitiven Bereiche eine maximale Länge von 7 cm auf. Besonders vorteilhaft weisen die einzelnen berührsensitiven und/oder drucksensitiven Bereiche eine maximale Länge von 5 cm auf. Besonders vorteilhaft weisen die einzelnen berührsensitiven und/oder drucksensitiven Bereiche eine maximale Länge von 3 cm auf. Die einzelnen berührsensitiven und/oder drucksensitiven Bereiche sind hierbei direkt aneinander angrenzend in Richtung der Längserstreckung des berührsensitiven und/oder drucksensitiven Positionseingabeelements nacheinander angeordnet.

Durch diese Ausgestaltung kann besonders einfach und exakt eine Position, insbesondere eine Untersuchungsposition, für die anstehende medizinische Bildgebungsuntersuchung von einem Benutzer, insbesondere einem medizinischen Bedienpersonal, an dem Patiententisch ausgewählt werden.

In einer vorteilhaften Weiterbildung der Patientenlagerungsvorrichtung kann es vorgesehen sein, dass die Positionseingabeeinheit ein erste Positionseingabeelement, das an einem ersten seitlichen Randbereich des Patiententischs angeordnet ist, und ein zweites Positionseingabeelement umfasst, das an einem zweiten seitlichen Randbereich des Patiententischs angeordnet ist, wobei der zweite Randbereich des Patiententischs zu dem ersten Randbereich des Patiententischs gegenüberliegend angeordnet ist. Vorzugsweise ist zwischen dem ersten Randbereich und dem zweiten Randbereich der Lagerungsbereich zur Lagerung des Patienten angeordnet. Hierdurch kann vorteilhaft an beiden Seitenbereichen des Patiententisch jeweils ein Positionseingabeelement der Positionseingabeeinheit angeordnet sein. Durch diese Ausgestaltung kann ein hoher Bedienkomfort der Positionseingabeeinheit für einen Benutzer bereitgestellt werden, da der Benutzer unabhängig von seiner Position am Patiententisch stets einen Zugriff auf die Positionseingabeeinheit hat.

In einer vorteilhaften Weiterbildung der Patientenlagerungsvorrichtung kann es vorgesehen sein, dass die Positionsausgabeeinheit eine Länge umfasst, die im Wesentlichen einer Länge des Patiententischs entspricht. Die Positionsausgabeeinheit kann dabei ein Positionsausgabeelement umfassen, wobei eine Länge des Positionsausgabeelements im Wesentlichen einer Länge des Patiententischs entspricht. Umfasst die Positionsausgabeeinheit mehrere Positionsausgabeelemente, die in Richtung einer Längserstreckung des Patiententischs nacheinander angeordnet sind, so entspricht im Wesentlichen eine Länge der mehreren Positionsausgabeelemente einer Länge des Patiententischs. Hierbei soll unter einer Länge, die im Wesentlichen einer Länge des Patiententischs entspricht, insbesondere verstanden werden, dass die Länge der Positionsausgabeeinheit mindestens 80 % der Länge des Patiententischs, insbesondere mindestens 80 % der Länge des Lagerungsbereichs des Patiententischs, entspricht. Besonders vorteilhaft entspricht die Länge der Positionsausgabeeinheit mindestens 85 % der Länge des Patiententischs, insbesondere mindestens 85 % der Länge des Lagerungsbereichs des Patiententischs. Besonders vorteilhaft entspricht die Länge der Positionsausgabeeinheit mindestens 90 % der Länge des Patiententischs, insbesondere mindestens 90 % der Länge des Lagerungsbereichs des Patiententischs. Besonders vorteilhaft entspricht die Länge der Positionsausgabeeinheit mindestens 95 % der Länge des Patiententischs, insbesondere mindestens 95 % der Länge des Lagerungsbereichs des Patiententischs. Besonders vorteilhaft entspricht die Länge der Positionsausgabeeinheit mindestens 98 % der Länge des Patiententischs, insbesondere mindestens 98 % der Länge des Lagerungsbereichs des Patiententischs.

Ein Vorteil dieser Ausgestaltung ist, dass die Ausgabe des Positionskontrollsignals mittels der Positionsausgabeeinheit an einer Position erfolgt, welche Position in Richtung einer Längserstreckung des Patiententischs mit einer Position der Positionseingabe mittels der Positionseingabeeinheit übereinstimmen kann. Hierdurch kann eine gute Erfassbarkeit des Feedbacks und/oder Positionskontrollsignals für den Benutzer ermöglicht und/oder gewährleistet werden.

In einer vorteilhaften Weiterbildung der Patientenlagerungsvorrichtung kann es vorgesehen sein, dass die Positionsausgabeeinheit ein Positionsausgabeelement mit zwei oder mehr Ausgabebereichen umfasst, die in Richtung einer Längserstreckung des zumindest einen Positionsausgabeelements nacheinander angeordnet sind. Vorzugsweise weisen die einzelnen Ausgabebereiche eine maximale Länge von 15 cm auf.

Besonders vorteilhaft weisen die einzelnen Ausgabebereiche eine maximale Länge von 10 cm auf. Besonders vorteilhaft weisen die einzelnen Ausgabebereiche eine maximale Länge von 7 cm auf. Besonders vorteilhaft weisen die einzelnen Ausgabebereiche eine maximale Länge von 5 cm auf. Besonders vorteilhaft weisen die einzelnen Ausgabebereiche eine maximale Länge von 3 cm auf. Die einzelnen Ausgabebereiche sind bevorzugt direkt aneinander angrenzend in Richtung der Längserstreckung des Positionsausgabeelement nacheinander angeordnet.

Durch diese Ausgestaltung kann eine besonders exakte und/oder genaue Ausgabe eines Feedbacks und/oder Positionskontrollsignals direkt am Ort und/oder der Position der Positionseingabe für den Benutzer erfolgen werden. Insbesondere können derart Fehlpositionierungen des Patienten für eine medizinische Bildgebungsuntersuchung von dem Benutzer direkt erkannt werden und damit auch reduziert werden. Zudem kann derart eine Vorbereitungszeit reduziert werden und damit ein Patientendurchsatz für eine medizinische Bildgebungsvorrichtung erhöht werden.

In einer vorteilhaften Weiterbildung der Patientenlagerungsvorrichtung kann es vorgesehen sein, dass die Positionsausgabeeinheit ein erstes Positionsausgabeelement, das an einem ersten seitlichen Randbereich des Patiententischs angeordnet ist, und ein zweiten Positionsausgabeelement umfasst, das an einem zweiten seitlichen Randbereich des Patiententischs angeordnet ist, wobei der zweite seitliche Randbereich des Patiententischs zu dem ersten seitlichen Randbereich des Patiententischs gegenüberliegend angeordnet ist. Vorzugsweise ist zwischen dem ersten seitlichen Randbereich und dem zweiten seitliche Randbereich der Lagerungsbereich zur Lagerung des Patienten angeordnet. Hierdurch kann an beiden seitlichen Randbereichen des Patiententisch jeweils ein Positionsausgabeelement der Positionsausgabeeinheit angeordnet sein. Dies ermöglicht einen hohen Bedienkomfort der Positionsausgabeeinheit für einen Benutzer, da dieser unabhängig von seiner Position am Patiententisch stets eine direkte und/oder uneingeschränkte Sicht auf eines der Positionsausgabeelemente hat.

In einer vorteilhaften Weiterbildung der Patientenlagerungsvorrichtung kann es vorgesehen sein, dass die Positionsausgabeeinheit zumindest ein optisches Positionsausgabeelement und/oder ein haptisches Positionsausgabeelement umfasst. Das zumindest eine optische Positionsausgabeelement kann beispielsweise zumindest ein LED-Element umfassen, das an dem seitlichen Randbereich des Patiententischs angeordnet ist. Das zumindest eine LED-Element kann dabei in einzelne LED-Leuchtbereiche unterteilt sein, so dass die einzelnen LED- Leuchtbereiche separat angesteuert werden können. Zudem kann das zumindest eine LED-Element auch einstückig und/oder einteilig mit einem Positionseingabeelement, insbesondere einem berührsensitiven und/oder drucksensitiven Positionseingabeelement, ausgebildet sein, so dass eine besonders platzsparende Positionierungseinheit bereitgestellt werden kann. Zudem sind weitere, dem Fachmann als sinnvoll erscheinende optische Positionsausgabeelemente jederzeit denkbar.

Das zumindest eine haptische Positionsausgabeelement kann dabei einzelne Vibrationsbereiche aufweisen. Bevorzugt ist hierbei das haptische Positionsausgabeelement einstückig und/oder einteilig mit dem Positionseingabeelement, insbesondere einem berührsensitiven und/oder drucksensitiven Positionseingabeelement, ausgebildet, so dass der Benutzer, insbesondere das medizinische Bedienpersonal, direkt bei der Eingabe der Position ein Feedback für die Eingabe erhält.

Die medizinische Bildgebungsvorrichtung umfasst bevorzugt eine Scannereinheit und einen Patientenaufnahmebereich. Die Scannereinheit umgibt zumindest teilweise den Patientenaufnahmebereich. Innerhalb des Patientenaufnahmebereichs ist der Patiententisch der Patientenlagerungsvorrichtung bewegbar angeordnet.

Bei einer Ausbildung der medizinischen Bildgebungsvorrichtung als Magnetresonanzvorrichtung umfasst die Scannereinheit typischerweise einen Grundmagneten. Der Grundmagnet ist zu einem Erzeugen eines starken, homogenen und konstanten Grundmagnetfelds ausgelegt, wobei der Grundmagnet derart ausgebildet ist, dass das starke, homogene und konstante Grundmagnetfeld in dem Patientenaufnahmebereich, insbesondere in einem Field of View (FOV), der Magnetresonanzvorrichtung angelegt ist. Des Weiteren umfasst die Scannereinheit bevorzugt eine Gradientenspuleneinheit und eine Hochfrequenzantenneneinheit.

Durch die Ausgestaltung kann vorteilhaft eine direkte Eingabe der Untersuchungsposition für eine Positionierung des Patiententischs innerhalb eines Isozentrums der medizinischen Bildgebungsvorrichtung erfolgen, ohne dass vorher der Patiententisch von einem Benutzer in eine definierte Position bewegt werden muss. Zudem kann auch direkt ein Feedback und/oder ein Positionskontrollsignal an den Benutzer, insbesondere an ein medizinisches Bedienpersonal, mittels der Positionsausgabeeinheit ausgegeben werden. Somit erhält der Benutzer auch direkt oder unmittelbar nach der Eingabe der Untersuchungsposition eine Bestätigung für seine Eingabe. Insbesondere kann die Ausgabe eines Feedbacks und/oder eines Positionskontrollsignals an den Benutzer an einem Ort und/oder einer Position erfolgen, die in Richtung einer Längserstreckung des Patiententischs mit einer Position der Positionseingabe mittels der Positionseingabeeinheit übereinstimmt, so dass die Ausgabe direkt im Sichtfeld des Benutzers erfolgen kann. Besonders vorteilhaft kann hierdurch ein hoher Bedienkomfort für den Benutzer während einer Vorbereitung des Patienten bereitgestellt werden. Weiterhin können derart insbesondere Fehlpositionierungen des Patienten innerhalb des Patientenaufnahmebereichs reduziert und/oder verhindert werden. Des Weiteren kann derart auch eine Vorbereitungszeit zur Vorbereitung des Patienten für die medizinische Bildgebungsuntersuchung reduziert werden und damit auch ein hoher Patientendurchsatz ermöglicht werden.

Die Vorteile der medizinischen Bildgebungsvorrichtung entsprechen im Wesentlichen den Vorteilen der Patientenlagerungsvorrichtung, welche vorab im Detail ausgeführt sind. Hierbei erwähnte Merkmale, Vorteile oder alternative Ausführungsformen können auch auf die anderen beanspruchten Gegenstände übertragen werden und umgekehrt.

Die Erfindung betrifft ein Verfahren zu einem Positionieren eines Patienten für eine medizinischen Bildgebungsuntersuchung, insbesondere eine Magnetresonanzuntersuchung, nach Anspruch 1.

Das Erfassen einer Untersuchungsposition mittels der Positionseingabeeinheit umfasst zunächst eine manuelle Eingabe der Untersuchungsposition mittels der Positionseingabeeinheit durch den Benutzer. Durch die Eingabe der Untersuchungsposition legt der Benutzer, insbesondere das medizinische Bedienpersonal, eine Position an dem Patiententisch fest, die während der medizinischen Bildgebungsuntersuchung im Isozentrum der medizinischen Bildgebungsvorrichtung angeordnet sein soll.

Für die Ausgabe des Positionskontrollsignals mittels der Positionsausgabeeinheit ist die Positionsausgabeeinheit vorzugsweise derart ausgebildet, dass die Ausgabe des Positionskontrollsignals am Ort und/oder der Position erfolgt, wobei der Ort und/oder die Position in Richtung einer Längserstreckung des Patiententischs mit einer Position der Positionseingabe mittels der Positionseingabeeinheit übereinstimmt. Hierzu können die Positionseingabeeinheit und die Positionsausgabeeinheit zumindest teilweise einstückig und/oder einteilig ausgebildet sein.

Die Patientenlagerungsvorrichtung, insbesondere die Positionierungseinheit der Patientenlagerungsvorrichtung, weist bevorzugt eine Recheneinheit auf, wobei die Recheneinheit die mittels der Positionseingabeeinheit erfasste Eingabe auswertet und ein entsprechendes und/oder auf die Eingabe abgestimmtes Ausgabesignal generiert. Das Ausgabesignal ist vorzugsweise von dem Positionskontrollsignal gebildet und wird von der Recheneinheit an die Positionsausgabeeinheit übertragen und dort an den Benutzer ausgegeben. Zudem kann mittels der Recheneinheit auch eine anschließende Bewegung des Patiententischs in Abhängigkeit der erfassten Untersuchungsposition gesteuert und/oder kontrolliert werden.

Eine derartige Recheneinheit der Positionierungseinheit kann zumindest ein Rechenmodul und/oder einen Prozessor umfassen, wobei die Recheneinheit zum Auswerten der mittels der Positionseingabeeinheit erfasste Eingabe und zum Generieren des Ausgabesignals ausgebildet ist. So ist insbesondere die Recheneinheit dazu ausgebildet, computerlesbare Instruktionen auszuführen, um das Auswerten der mittels der Positionseingabeeinheit erfasste Eingabe und das Generieren des Ausgabesignals erfindungsgemäße Verfahren auszuführen. Insbesondere umfasst die Recheneinheit eine Speichereinheit, wobei auf der Speichereinheit computerlesbare Informationen gespeichert sind, wobei die Recheneinheit dazu ausgebildet ist, die computerlesbaren Informationen von der Speichereinheit zu laden und die computerlesbaren Informationen auszuführen.

Die Komponenten der Recheneinheit können dabei zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Das Positionskontrollsignal kann beispielsweise ein optisches Positionskontrollsignal umfassen, das mittels einer optischen Positionsausgabeeinheit an den Benutzer ausgebbar ist. Alternativ oder zusätzlich kann das Positionskontrollsignal ein haptisches Positionsprotokollsignal umfassen, das mittels einer haptischen Positionsausgabeeinheit an den Benutzer ausgebbar ist.

Durch die erfindungsgemäße Ausgestaltung des Verfahrens zu einem Positionieren eines Patienten für eine medizinische Bildgebungsuntersuchung kann vorteilhaft eine direkte Eingabe der Untersuchungsposition für eine Positionierung des Patiententischs innerhalb eines Isozentrums der medizinischen Bildgebungsvorrichtung erfolgen, ohne dass vorher der Patiententisch von einem Benutzer in eine definierte Position bewegt werden muss. Zudem kann auch direkt ein Feedback und/oder ein Positionskontrollsignal an den Benutzer, insbesondere an ein medizinisches Bedienpersonal, mittels der Positionsausgabeeinheit ausgegeben werden. Somit erhält der Benutzer auch direkt oder unmittelbar nach der Eingabe der Untersuchungsposition eine Bestätigung für seine Eingabe. Insbesondere kann die Ausgabe eines Feedbacks und/oder eines Positionskontrollsignals an den Benutzer an einem Ort und/oder einer Position erfolgen, die in Richtung einer Längserstreckung des Patiententischs mit einer Position der Positionseingabe mittels der Positionseingabeeinheit übereinstimmt, so dass die Ausgabe direkt im Sichtfeld des Benutzers erfolgen kann. Besonders vorteilhaft kann hierdurch ein hoher Bedienkomfort für den Benutzer während einer Vorbereitung des Patienten bereitgestellt werden. Weiterhin können derart insbesondere Fehlpositionierungen des Patienten innerhalb des Patientenaufnahmebereichs reduziert und/oder verhindert werden. Des Weiteren kann derart auch eine Vorbereitungszeit zur Vorbereitung des Patienten für die medizinische Bildgebungsuntersuchung reduziert werden und damit auch ein hoher Patientendurchsatz ermöglicht werden.

Die Vorteile des Verfahrens zu einem Positionieren eines Patienten für eine medizinische Bildgebungsuntersuchung entsprechen im Wesentlichen den Vorteilen der Patientenlagerungsvorrichtung bzw. der erfindungsgemäßen medizinischen Bildgebungsvorrichtung, welche vorab im Detail ausgeführt sind. Hierbei erwähnte Merkmale, Vorteile oder alternative Ausführungsformen können ebenso auf die anderen beanspruchten Gegenstände übertragen werden und umgekehrt.

In einer vorteilhaften Weiterbildung des erfindungsgemäßen Verfahrens kann es vorgesehen sein, dass das Erfassen der Untersuchungsposition ein Betätigen eines Positionseingabeelements der Positionseingabeeinheit an einer Position umfasst, wobei die Position in Richtung einer Längserstreckung des Patiententischs mit dem zu untersuchenden Bereich des Patienten übereinstimmt. Das Betätigen eines Positionseingabeelements umfasst bevorzugt ein Berühren und/oder ein Drücken des Positionseingabeelements. Vorzugsweise umfasst das Positionseingabeelements ein berührsensitives und/oder drucksensitives Positionseingabeelement mit einzelnen berührsensitiven und/oder drucksensitiven Bereichen. Die einzelnen berührsensitiven und/oder drucksensitiven Bereiche sind bevorzugt in Längserstreckung des Patiententischs und/oder in Längserstreckung des Positionseingabeelements nacheinander, insbesondere aneinander angrenzend, angeordnet.

Hierdurch kann eine besonders einfache und intuitive Eingabe der Untersuchungsposition für einen Benutzer, insbesondere ein medizinisches Bedienpersonal, ermöglicht werden. Dabei kann ein Benutzer direkt bei einer Vorbereitung des zu untersuchenden Bereichs des Patienten, beispielsweise durch Anordnen von für die anstehende medizinische Bildgebungsuntersuchung erforderlichen Zubehöreinheiten um den zu untersuchenden Bereich, wie beispielsweise ein Anordnen einer lokalen Hochfrequenzantenneneinheit, direkt die Untersuchungsposition an der Positionseingabeeinheit eingeben, ohne hierbei seine Position zu wechseln.

Es ist vorgesehen, dass für eine medizinische Bildgebungsuntersuchung n Untersuchungspositionen mittels der Positionseingabeeinheit eingebbar sind, wobei eine n-te Untersuchungsposition durch ein n-faches Betätigen der Positionseingabeeinheit festgelegt wird. Für medizinische Bildgebungsuntersuchungen, bei denen von unterschiedliche Körperbereich des Patienten medizinische Bilddaten erfasst werden, ist es erforderlich, dass der Patient für einzelne Messabschnitte der medizinischen Bildgebungsuntersuchung an unterschiedliche Positionen, insbesondere Untersuchungspositionen, innerhalb des Patientenaufnahmebereichs der medizinischen Bildgebungsvorrichtung positioniert ist, um den für die einzelnen Messabschnitte relevanten Körperbereiche im Isozentrum der medizinischen Bildgebungsvorrichtung zu positionieren. Diese unterschiedlichen Untersuchungspositionen für eine einzige medizinische Bildgebungsuntersuchung können hierbei von dem Benutzer, insbesondere dem medizinischen Bedienpersonal, während der Vorbereitung des Patienten mittels der Positionseingabeeinheit eingegeben werden. Hierbei wird die n-te Untersuchungsposition durch ein n-faches Betätigen des Positionseingabeeinheit festgelegt. Insbesondere wird hierbei die n-fache Eingabe der Untersuchungsposition an demselben Eingabebereich, beispielsweise an demselben drucksensitiven Bereich eines Positionseingabeelements, getätigt. So wird die erste Untersuchungsposition durch ein einmaliges Betätigen des Eingabebereichs des Positionseingabeelements von dem Benutzer festgelegt. Eine zweite Untersuchungsposition wird durch ein zweimaliges Betätigen des Eingabebereichs des Positionseingabeelements von dem Benutzer festgelegt. Eine dritte Untersuchungsposition wird durch ein dreimaliges Betätigen des Eingabebereichs des Positionseingabeelements von dem Benutzer festgelegt, usw. Zudem kann es auch vorgesehen sein, dass beispielsweise eine zweite Untersuchungsposition durch ein Betätigen des Eingabebereichs des Positionseingabeelements mit beispielsweis zwei Fingern festgelegt wird.

Durch diese Ausgestaltung kann eine besonders einfache und intuitive Eingabe von mehreren Untersuchungspositionen für einen Benutzer bereitgestellt werden. Insbesondere kann derart ein hoher Bedienkomfort während einer Vorbereitung eines Patienten für eine medizinische Bildgebungsuntersuchung erreicht werden. Zudem kann derart auch eine zeitsparende Eingabe und/oder Erfassung von mehreren Untersuchungspositionen erfolgen.

In einer vorteilhaften Weiterbildung des erfindungsgemäßen Verfahrens kann es vorgesehen sein, dass das Erfassen der Untersuchungsposition eine Eingabe eines Untersuchungsbereichs umfasst, wobei die Eingabe des Untersuchungsbereichs ein Festlegen einer Bereichsstartposition durch Kontaktierung des Benutzers mit einem ersten Eingabebereich der Positionseingabeeinheit und einer kontinuierlichen Kontaktierung der Positionseingabeeinheit bis zu einem Erreichen einer Bereichsendposition umfasst. Vorzugsweise wird hierbei die Bereichsstartposition an einem ersten Eingabebereich der Positionseingabeeinheit, insbesondere eines Positionseingabeelements, festgelegt. Die kontinuierliche Kontaktierung der Positionseingabeeinheit, insbesondere des Positionseingabeelements, erfolgt bevorzugt in Richtung einer Längserstreckung des Patiententischs und/oder in Richtung einer Längserstreckung des Positionseingabeelements durch den Benutzer bis zur Bereichsendposition. Dabei kann der Benutzer ein oder mehrere Eingabebereiche, die zwischen der Bereichsstartposition und der Bereichsendposition angeordnet sind, kontaktiert, beispielsweise durch eine Wischbewegung mit einem Finger an der Oberfläche des Positionseingabeelements von der Bereichsstartposition zu der Bereichsendposition. Die Bereichsendposition wird durch einen Eingabebereich der Positionseingabeeinheit, insbesondere des Positionseingabeelements, festgelegt, an dem die letzte Kontaktierung des Benutzers mit der Positionseingabeeinheit, insbesondere dem Positionseingabeelement, erfolgt ist. Vorzugsweise ist der Eingabebereich der Bereichsendposition unterschiedlich zu dem Eingabebereich der Bereichsstartposition.

Hierdurch kann eine besonders zeitsparende Eingabe eines Untersuchungsbereich erfolgen und damit auch ein hoher Patientendurchsatz einer medizinischen Bildgebungsvorrichtung ermöglicht werden.

In einer vorteilhaften Weiterbildung des erfindungsgemäßen Verfahrens kann es vorgesehen sein, dass die Ausgabe des Positionskontrollsignals an einer Position erfolgt, welche Position in Richtung einer Längserstreckung des Patiententischs mit einer Position der Positionseingabe mittels der Positionseingabeeinheit übereinstimmt. Vorzugsweise erfolgt die Ausgabe des Positionskontrollsignals unmittelbar nach der Erfassung und/oder Eingabe der Untersuchungsposition mittels der Positionseingabeeinheit. Für eine derartige Ausgestaltung des erfindungsgemäßen Verfahrens können die Positionseingabeeinheit und die Positionsausgabeeinheit einstückig und/oder einteilig miteinander ausgebildet sein. Hierdurch kann ein Benutzer, insbesondere ein medizinisches Bedienpersonal, direkt die eingegebene Position für die Positionierung kontrollieren. Weiterhin können derart insbesondere Fehlpositionierungen reduziert und/oder verhindert werden. Des Weiteren kann derart auch eine Vorbereitungszeit zur Vorbereitung des Patienten für die medizinische Bildgebungsuntersuchung reduziert werden.

In einer vorteilhaften Weiterbildung des erfindungsgemäßen Verfahrens kann es vorgesehen sein, dass die Ausgabe des Positionskontrollsignals eine Ausgabe eines optischen Positionskontrollsignals und/oder eines haptischen Positionskontrollsignals umfasst. Vorzugsweise weist hierbei die Positionsausgabeeinheit ein optisches Positionsausgabeelement und/oder ein haptisches Positionsausgabeelement auf. Beispielseise kann das optische Positionsausgabeelement mehrere LED-Elemente umfassen, die einzelnen Bereiche des Positionsausgabeelements ausleuchten können. Zudem können die einzelnen Bereiche auch mit unterschiedlichen Farben ausgeleuchtet werden. Das haptische Positionsausgabeelement kann beispielsweise ein Vibrationselement umfassen, mittels dessen einzelnen Ausgabebereich des Positionsausgabeelements zur Vibration gebracht werden können.

In einer vorteilhaften Weiterbildung des erfindungsgemäßen Verfahrens kann es vorgesehen sein, dass der Patiententisch für eine Eingabe der zumindest einen Untersuchungsposition in einer Home-Position außerhalb des Patientenaufnahmebereich der medizinischen Bildgebungsvorrichtung positioniert ist, wobei die Patienteneingabeeinheit deaktiviert wird, sobald der Patiententisch die Home-Position verlässt. Während einer Vorbereitung des Patienten für die anstehende medizinische Bildgebungsuntersuchung ist der Patiententisch der Patientenlagerungsvorrichtung in einer Home-Position angeordnet. In dieser Home-Position befindet sich der Patiententisch außerhalb des Patientenaufnahmebereichs, um eine gute Zugänglichkeit zum Patienten während der Vorbereitung für einen Benutzer, insbesondere das medizinische Bedienpersonal, zu gewährleisten. Für eine medizinische Bildgebungsuntersuchung muss der Patient, insbesondere der zu untersuchende Bereich des Patienten, innerhalb des Patientenaufnahmebereichs, insbesondere des Isozentrums, angeordnet werden. Hierzu wird der Patiententisch nach der Vorbereitung des Patienten von der Home-Position in den Patientenaufnahmebereich gefahren und/oder bewegt.

Die Deaktivierung der Patienteneingabeeinheit, insbesondere der einzelnen Patienteneingabeelemente der Patienteneingabeeinheit, erfolgt bevorzugt automatisch und/oder selbsttätig mittels einer Recheneinheit und/oder Steuerungseinheit der Patientenlagerungsvorrichtung.

Durch diese Ausgestaltung des Verfahrens kann vorteilhaft verhindert werden, dass durch eine Bewegung des Patienten während der medizinischen Bildgebungsuntersuchung versehentlich eine neue Untersuchungsposition eingestellt wird. Dabei kann auch eine hohe Sicherheit für den Patienten während der medizinischen Bildgebungsuntersuchung bereitgestellt werden.

In einer vorteilhaften Weiterbildung des erfindungsgemäßen Verfahrens kann es vorgesehen sein, dass eine Bewegungsgeste des Benutzers in eine einfahrende Richtung an der Positionseingabeeinheit erfasst wird, wobei die erfasste Bewegungsgeste in die einfahrende Richtung eine Einfahrbewegung des Patiententischs in einen Patientenaufnahmebereich startet. Eine Erfassung der Bewegungsgeste erfolgt bevorzugt durch Kontaktierung von beispielsweise einer Hand und/oder eines Fingers des Benutzers, insbesondere des medizinischen Bedienpersonals, mit der Positionseingabeeinheit, insbesondere einem Positionseingabeelement. Die Einfahrbewegung erfolgt bevorzugt von der Home-Position des Patiententischs in den Patientenaufnahmebereich hinein. Bevorzugt weist auch die Bewegungsgeste in einfahrender Richtung von der Home-Position des Patiententischs in den Patientenaufnahmebereich hinein.

Durch diese Ausgestaltung des Verfahrens kann besonders einfach eine Einfahrbewegung des Patiententischs in den Patientenaufnahmebereich hinein durch einen Benutzer gestartet werden. Insbesondere kann derart der Benutzer ein Startsignal an einer beliebigen Position an dem Patiententischs geben für ein Einfahren des Patiententischs und muss hierzu nicht seine Position und/oder Standort ändern, um an einen Starttaste zum Einfahren des Patiententischs zu gelangen.

Weitere Vorteile, Merkmale und Einzelheiten der Erfindung ergeben sich aus dem im Folgenden beschriebenen Ausführungsbeispiel sowie anhand der Zeichnungen.

Es zeigen:
- Fig. 1: eine medizinische Bildgebungsvorrichtung in einer schematischen Darstellung,
- Fig. 2: eine Patiententisch einer Patientenlagerungsvorrichtung in einer Draufsicht,
- Fig. 3: eine Eingabe eines Untersuchungsbereichs an dem Patiententisch,
- Fig. 4: ein Verfahren zu einem Positionieren eines Patienten für eine medizinische Bildgebungsuntersuchung mittels einer Patientenlagerungsvorrichtung und
- Fig. 5: eine alternative Ausgestaltung einer Positionierungseinheit eines Patiententischs.

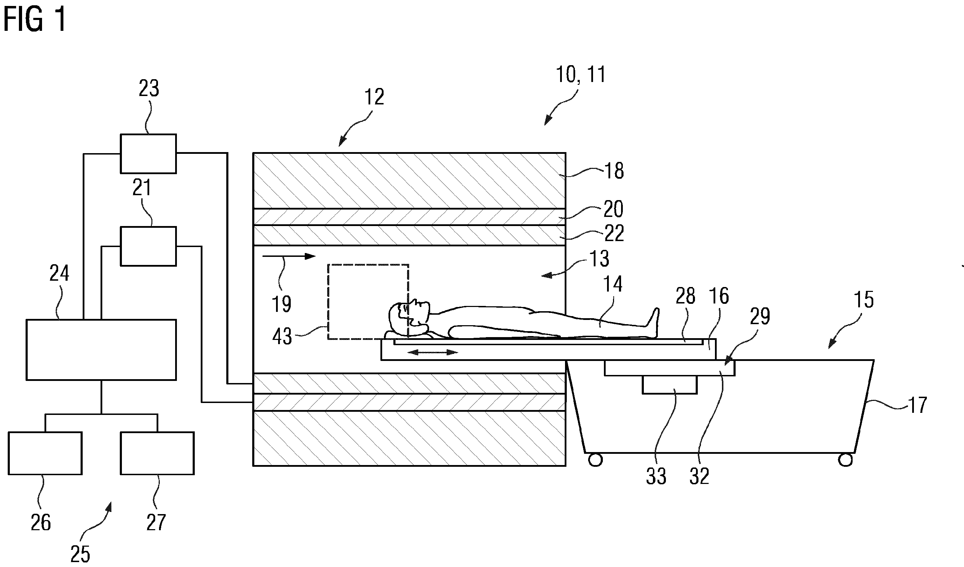

In der Fig. 1 ist eine medizinische Bildgebungsvorrichtung 10 schematisch dargestellt. Die medizinische Bildgebungsvorrichtung 10 ist im vorliegenden Ausführungsbeispiel von einer Magnetresonanzvorrichtung 11 gebildet, wobei beispielhaft die vorliegende Erfindung anhand der Magnetresonanzvorrichtung 11 erläutert wird. Die vorliegende Erfindung ist jedoch nicht auf Ausgestaltung der medizinischen Bildgebungsvorrichtung 10 auf eine Magnetresonanzvorrichtung 11 beschränkt und weitere Ausgestaltungen der medizinischen Bildgebungsvorrichtung 10 sind jederzeit denkbar.

Die Magnetresonanzvorrichtung 11 umfasst eine von einer Magneteinheit gebildeten Scannereinheit 12. Zudem weist die Magnetresonanzvorrichtung 11 einen Patientenaufnahmebereich 13 auf zu einer Aufnahme eines Patienten 14. Der Patientenaufnahmebereich 13 im vorliegenden Ausführungsbeispiel ist zylinderförmig ausgebildet und in einer Umfangsrichtung von der Scannereinheit 12, insbesondere von der Magneteinheit, zylinderförmig umgeben. Grundsätzlich ist jedoch eine davon abweichende Ausbildung des Patientenaufnahmebereichs 13 jederzeit denkbar.

Der Patient 14 kann mittels einer Patientenlagerungsvorrichtung 15 der Magnetresonanzvorrichtung 11 in den Patientenaufnahmebereich 13 geschoben und/oder gefahren werden. Die Patientenlagerungsvorrichtung 15 weist hierzu einen bewegbar ausgestalteten Patiententisch 16 auf. Insbesondere ist hierbei der Patiententisch 16 in Richtung einer Längserstreckung des Patientenaufnahmebereichs 13 und/oder in z-Richtung bewegbar gelagert. Die Patientenlagerungsvorrichtung 15 umfasst bevorzugt eine Basiseinheit 17, wobei der Patiententisch 16 der Patientenlagerungsvorrichtung 15 bezüglich der Basiseinheit 17 bewegbar ausgebildet ist.

Die Scannereinheit 12, insbesondere die Magneteinheit, umfasst einen supraleitenden Grundmagneten 18 zu einem Erzeugen eines starken und insbesondere konstanten Grundmagnetfelds 19. Weiterhin weist die Scannereinheit 12, insbesondere die Magneteinheit, eine Gradientenspuleneinheit 20 zu einer Erzeugung von Magnetfeldgradienten auf, die für eine Ortskodierung während einer Bildgebung verwendet werden. Die Gradientenspuleneinheit 20 wird mittels einer Gradientensteuereinheit 21 der Magnetresonanzvorrichtung 11 gesteuert. Die Scannereinheit 12, insbesondere die Magneteinheit, umfasst weiterhin eine Hochfrequenzantenneneinheit 22 zu einer Anregung einer Polarisation, die sich in dem von dem Grundmagneten 17 erzeugten Grundmagnetfeld 18 einstellt. Die Hochfrequenzantenneneinheit 22 wird von einer Hochfrequenzantennensteuereinheit 23 der Magnetresonanzvorrichtung 11 gesteuert und strahlt hochfrequente Magnetresonanzsequenzen in den Patientenaufnahmebereich 13 der Magnetresonanzvorrichtung 11 ein.

Zu einer Steuerung des Grundmagneten 18, der Gradientensteuereinheit 21 und zur Steuerung der Hochfrequenzantennensteuereinheit 23 weist die Magnetresonanzvorrichtung 11 eine Systemsteuereinheit 24 auf. Die Systemsteuereinheit 24 steuert zentral die Magnetresonanzvorrichtung, wie beispielsweise das Durchführen einer vorbestimmten bildgebenden Gradientenechosequenz. Zudem umfasst die Systemsteuereinheit 24 eine nicht näher dargestellte Auswerteeinheit zu einer Auswertung von medizinischen Bilddaten, die während der Magnetresonanzuntersuchung erfasst werden.

Des Weiteren umfasst die Magnetresonanzvorrichtung 11 eine Benutzerschnittstelle 25, die mit der Systemsteuereinheit 24 verbunden ist. Steuerinformationen wie beispielsweise Bildgebungsparameter, sowie rekonstruierte Magnetresonanzbilder können auf einer Anzeigeeinheit 26, beispielsweise auf zumindest einem Monitor, der Benutzerschnittstelle 25 für ein medizinisches Bedienpersonal angezeigt werden. Weiterhin weist die Benutzerschnittstelle 25 eine Eingabeeinheit 27 auf, mittels der Informationen und/oder Parameter während eines Messvorgangs von dem medizinischen Bedienpersonal eingegeben werden können.

Die dargestellte medizinische Bildgebungsvorrichtung 10, insbesondere die Magnetresonanzvorrichtung 11, kann selbstverständlich weitere Komponenten umfassen, die medizinische Bildgebungsvorrichtungen 10, insbesondere die Magnetresonanzvorrichtungen 11, gewöhnlich aufweisen. Eine allgemeine Funktionsweise einer medizinischen Bildgebungsvorrichtung 10, insbesondere der Magnetresonanzvorrichtung 11, ist zudem dem Fachmann bekannt, so dass auf eine detaillierte Beschreibung der weiteren Komponenten verzichtet wird.

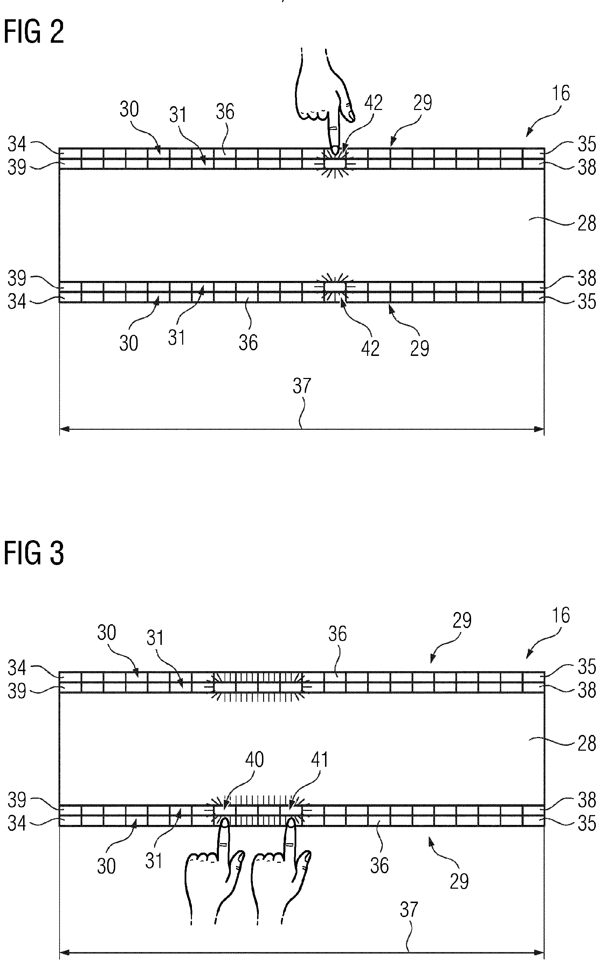

Der Patiententisch 16 der Patientenlagerungsvorrichtung 15 ist in den Fig. 2, 3 und 5 in einer Draufsicht dargestellt. Der Patiententisch 16 weist einen Lagerungsbereich 28 zu einer Aufnahme und/oder Lagerung von Patienten 14 auf. Für eine medizinische Bildgebungsuntersuchung, insbesondere eine Magnetresonanzuntersuchung ist der Patient 14 auf dem Lagerungsbereich 28 des Patiententischs 16 positioniert.

Die Patientenlagerungsvorrichtung 15 weist neben dem Patiententisch 16 eine Positionierungseinheit 29 auf. Die Positionierungseinheit 29 ist zu einer Positionierung des Patiententischs 16 innerhalb des Patientenaufnahmebereichs 13 der medizinischen Bildgebungsvorrichtung 10, insbesondere der Magnetresonanzvorrichtung 11, ausgebildet. Die Positionierungseinheit 29 weist eine Positionseingabeeinheit 30 und eine Positionsausgabeeinheit 31 auf. Des Weiteren weist die Positionierungseinheit 29 eine Antriebseinheit 32 auf zur Generierung einer Antriebsenergie und/oder eines Antriebsmoments zur Bewegung und/oder Positionierung des Patiententischs 16. Des Weiteren umfasst die Positionierungseinheit 29 auch eine Recheneinheit 33 zur Steuerung der Bewegung und/oder Positionierung des Patiententischs 16 (Fig. 1). Alternativ oder zusätzlich kann die Steuerung des Patiententisch 16 auch mittels einer Steuerungseinheit und/oder Recheneinheit der medizinischen Bildgebungsvorrichtung 10 erfolgen.

Mittels der Positionierungseinheit 29 erfolgt eine Positionierung des Patiententischs 16 und/oder eines zu untersuchenden Bereichs des Patienten 14 in einem FOV 43 und/oder einem Isozentrum der medizinischen Bildgebungsvorrichtung 10 insbesondere der Magnetresonanzvorrichtung 11.

Die Positionseingabeeinheit 30 ist an dem Patiententisch 16 angeordnet. Der Patiententisch 16 weist einen seitlichen Randbereich 34 auf, an dem die Positionseingabeeinheit 30 angeordnet ist. Der Patiententisch 16 weist zwei seitliche Randbereiche 34 auf. Die beiden seitlichen Randbereiche 34 sind an gegenüberliegenden Randbereichen des Patiententischs 16 angeordnet, so dass eine Lagerungsfläche des Lagerungsbereichs 28 zwischen den beiden seitlichen Randbereichen 34 angeordnet ist.

Die Positionseingabeeinheit 30 weist ein erstes Positionseingabeelement 35 und ein zweites Positionseingabeelement 35 auf, wobei das erste Positionseingabeelement 35 an dem ersten seitlichen Randbereich 34 und das zweite Positionseingabeelement 35 an dem zweiten seitlichen Randbereich 34 angeordnet ist. Die beiden Positionseingabeelemente 35 sind gleichartig ausgebildet.

Die beiden Positionseingabeelemente 35 weisen jeweils eine Länge auf, die im Wesentlichen einer Länge des Patiententischs 16 entspricht.

Im vorliegenden Ausführungsbeispiel sind die beiden Positionseingabeelemente 35 der Positionseingabeeinheit 30 jeweils als berührsensitives und/oder drucksensitives Positionseingabeelement 35 ausgebildet. Ein derartiges berührsensitives und/oder drucksensitives Positionseingabeelement 35 weist bevorzugt zwei oder mehr berührsensitive und/oder drucksensitive Eingabebereiche 36 auf, wobei die einzelnen berührsensitive und/oder drucksensitiven Bereiche in Richtung einer Längserstreckung 37 des Positionseingabeelements 35 nacheinander angeordnet sind. Bevorzugt sind in Richtung der Längserstreckung 37 des berührsensitiven und/oder drucksensitiven Positionseingabeelements 35 die einzelnen berührsensitiven und/oder drucksensitiven Eingabebereiche 36 direkt aneinander angrenzend angeordnet, so dass für jede beliebige Position entlang der Längserstreckung 37 des berührsensitiven und/oder drucksensitiven Positionseingabeelements 35 eine Positionseingabe für einen Benutzer, insbesondere das medizinische Bedienpersonal, möglich ist. Vorzugsweise weisen die einzelnen berührsensitiven und/oder drucksensitiven Eingabebereiche 36 eine maximale Länge von 15 cm auf. Besonders vorteilhaft weisen die einzelnen berührsensitiven und/oder drucksensitiven Eingabebereiche 36 eine maximale Länge von 10 cm auf. Besonders vorteilhaft weisen die einzelnen berührsensitiven und/oder drucksensitiven Eingabebereiche 36 eine maximale Länge von 7 cm auf. Besonders vorteilhaft weisen die einzelnen berührsensitiven und/oder drucksensitiven Eingabebereiche 36 eine maximale Länge von 5 cm auf. Besonders vorteilhaft weisen die einzelnen berührsensitiven und/oder drucksensitiven Eingabebereiche 36 eine maximale Länge von 3 cm auf.

Die Positionsausgabeeinheit 31 ist an dem Patiententisch 16 angeordnet. Insbesondere umfasst die Positionsausgabeeinheit 31 zwei Positionsausgabeelemente 38, wobei das erste Positionsausgabeelement 38 an dem ersten seitlichen Randbereich 34 und das zweite Positionsausgabeelement 38an dem zweiten seitlichen Randbereich 34 angeordnet ist. Die beiden Positionsausgabeelemente 38 sind gleichartig ausgebildet. Die beiden Positionsausgabeelemente 38 weisen jeweils eine Länge auf, die im Wesentlichen einer Länge des Patiententischs 16 entspricht.

Im vorliegenden Ausführungsbeispiel sind die beiden Positionsausgabeelemente 38 der Positionsausgabeeinheit 31 jeweils als optisches Positionsausgabeelement 38 und/oder als haptisches Positionsausgabeelement 38 ausgebildet. Ein derartiges Positionsausgabeelement 38 weist bevorzugt zwei oder mehr Ausgabebereiche 39 auf, wobei die einzelnen Ausgabebereiche 39 in Richtung einer Längserstreckung 37 des Patiententischs 16 nacheinander angeordnet sind. Bevorzugt sind in Richtung der Längserstreckung 37 des Patiententischs 16 die einzelnen Ausgabebereiche 39 direkt aneinander angrenzend angeordnet, so dass für jede beliebige Position entlang der Längserstreckung 37 des Patiententischs 16 eine Positionsausgabe an einen Benutzer, insbesondere das medizinische Bedienpersonal, erfolgen kann. Vorzugsweise weisen die einzelnen Ausgabebereiche 39 eine maximale Länge von 15 cm auf. Besonders vorteilhaft weisen die einzelnen Ausgabebereiche 39 eine maximale Länge von 10 cm auf. Besonders vorteilhaft weisen die einzelnen Ausgabebereiche 39 eine maximale Länge von 7 cm auf. Besonders vorteilhaft weisen die einzelnen Ausgabebereiche 39 eine maximale Länge von 5 cm auf. Besonders vorteilhaft weisen die einzelnen Ausgabebereiche 39 eine maximale Länge von 3 cm auf.

Die einzelnen Ausgabebereiche 39 der Positionsausgabeelemente 38 können hierbei optische Ausgabebereiche 39 umfassen, wie beispielsweise beleuchtbare und/oder hinterleuchtbare Ausgabebereiche 39. Insbesondere können hierbei die einzelnen Ausgabebereiche 39 jeweils ein oder mehrere LEDs umfassen für eine optische Ausgabe. Alternativ oder zusätzlich können die einzelnen Ausgabebereiche 39 der Positionsausgabeelemente 38 haptische Ausgabebereiche 39 umfassen, wie beispielsweise vibrierende Ausgabebereiche. Insbesondere können hierbei die einzelnen Ausgabebereiche 39 jeweils ein Vibrationselement umfassen, wie beispielsweise ein Unwuchtelement.

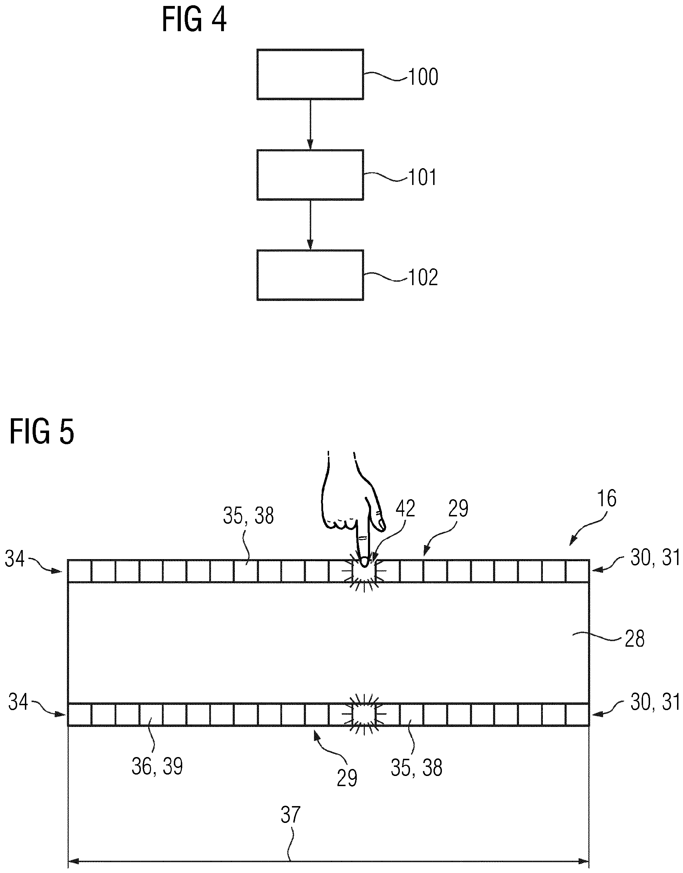

In Fig. 2 und 3 sind ist die Positionseingabeeinheit 30 separat zur Positionsausgabeeinheit 31 ausgebildet. Insbesondere sind die Positionseingabeeinheit 30 und die Positionsausgabeeinheit 31 als separate Einheiten ausgebildet. Dabei sind die Positionseingabeelemente 35 separat zu den Positionsausgabeelementen 38 ausgebildet. In Fig. 5 dagegen sind die Positionseingabeeinheit 30 und die Positionsausgabeeinheit 31 einstückig und/oder einteilig ausgebildet. Hierbei sind die Positionseingabeelemente 35 einstückig und/oder einteilig mit den Positionsausgabeelementen 38 ausgebildet.

In Fig. 4 ist ein Verfahren zu einem Positionieren eines Patienten 14 für eine medizinische Bildgebungsuntersuchung mittels der oben beschriebenen Patientenlagerungsvorrichtung 15 schematisch dargestellt. Das Verfahren zum Positionieren des Patienten 14 für eine medizinische Bildgebungsuntersuchung wird zumindest teilweise von der Recheneinheit 33 der Patientenlagerungsvorrichtung 15 gesteuert. Hierzu weist die Recheneinheit 33 eine Steuerungssoftware und/oder Steuerungsprogramme auf, die in einer nicht näher dargestellten Speichereinheit der Recheneinheit 33 gespeichert sind. Die Steuerungssoftware und/oder Steuerungsprogramme sind dazu ausgebildet, bei einem Ausführen der Steuerungssoftware und/oder Steuerungsprogramme durch einen Prozessor der Recheneinheit das Verfahren zu einem Positionieren des Patienten 14 für eine medizinische Bildgebungsuntersuchung zu steuern.

In einem ersten Verfahrensschritt 100 erfolgt ein Erfassen einer Untersuchungsposition 42 mittels einer Positionseingabeeinheit 30 der Patientenlagerungsvorrichtung 15, insbesondere des Patiententischs 16. Aufgrund der Ausgestaltung der Positionseingabeeinheit 30 kann dabei von einem Benutzer die Untersuchungsposition 42 an einer Position an der Positionseingabeeinheit 30 eingegeben werden, wobei die Position in Richtung der Längserstreckung 37 des Patiententischs 16 mit dem zu untersuchenden Bereich des Patienten 14 übereinstimmt.

Des Weiteren werden in diesem Verfahrensschritt 100 des Erfassens einer Untersuchungsposition 42 auch n Untersuchungspositionen 42 für eine medizinische Bildgebungsuntersuchung, insbesondere eine Magnetresonanzuntersuchung, von einem medizinischen Bedienpersonal mittels der Positionseingabeeinheit 30 eingegeben. Hierbei wird eine n-te Untersuchungsposition 42 durch ein n-faches Betätigen der Positionseingabeeinheit 30, insbesondere eines Eingabebereichs 36 eines der Positionseingabeelemente 35, festgelegt. Beispielsweise wird hierbei die Eingabe der Untersuchungsposition 42 an demselben Eingabebereich 36, beispielsweise an demselben drucksensitiven Eingabebereich 36, eines Positionseingabeelements, von dem medizinischen Bedienpersonal getätigt. So wird die erste Untersuchungsposition 42 durch ein einfaches Betätigen des Eingabebereichs 36 des Positionseingabeelements 35 festgelegt, wobei der Eingabebereich 36 mit dem ersten, zu untersuchenden Bereich des Patienten 14 in einer Richtung einer Längserstreckung 37 des Patiententischs 16 übereinstimmt. Eine zweite Untersuchungsposition 42 wird durch ein zweimaliges Betätigen des Eingabebereichs 36 des Positionseingabeelements 35 festgelegt, wobei der Eingabebereich 36 mit einem zweiten, zu untersuchenden Bereich des Patienten 34 in einer Richtung einer Längserstreckung 37 des Patiententischs 16 übereinstimmt. Eine dritte Untersuchungsposition 42 wird durch ein dreimaliges Betätigen des Eingabebereichs 36 des Positionseingabeelements 35 festgelegt, wobei der Eingabebereich 36 mit einem dritten, zu untersuchenden Bereich des Patienten 14 in einer Richtung einer Längserstreckung 37 des Patiententischs 16 übereinstimmt usw. Zudem kann es auch vorgesehen sein, dass beispielsweise eine zweite Untersuchungsposition 42 durch ein Betätigen des Eingabebereichs 36 des Positionseingabeelements 35 mit beispielsweis zwei Fingern festgelegt wird.

Zudem kann der Verfahrensschritt 100 des Erfassens einer Untersuchungsposition 42 auch eine Eingabe eines Untersuchungsbereichs umfassen, wie dies in Fig. 3 dargestellt ist. Der Untersuchungsbereich wird durch eine Eingabe und/oder ein Festlegen einer Bereichsstartposition 40 und einer Bereichsendposition 41 von dem medizinischen Bedienpersonal festgelegt. Hierbei wird insbesondere die Bereichsstartposition 40 durch Kontaktierung, beispielsweise ein Drücken und/oder Berühren, eines Eingabebereichs 36 der Positionseingabeeinheit 30 durch das medizinische Bedienpersonal festgelegt. Von der Bereichsstartposition 40 und/oder des kontaktierten Eingabebereichs 36 erfolgt anschließend eine kontinuierliche Kontaktierung der Positionseingabeeinheit 30 bis zu einem Erreichen der Bereichsendposition 41. Hierbei werden von dem medizinische Bedienpersonal alle Eingabebereiche 36, die zwischen der Bereichsstartposition 40 und der Bereichsendposition 41 angeordnet sind, kontaktiert, beispielsweise berührt und oder gedrückt.

Durch ein Betätigen eines Eingabebereichs 36 oder mehrere Eingabebereiche 36 eines der Positionseingabeelemente 35 der Positionseingabeeinheit 30 wird von der Recheneinheit 33 ein entsprechendes Eingabesignal erfasst, wobei die Recheneinheit 33mit der Positionseingabeeinheit 30 verbunden ist.

Anschließend erfolgt in einem zweiten Verfahrensschritt 101 eine Ausgabe eines Positionskontrollsignal mittels der Positionsausgabeeinheit 31 der Patientenlagerungsvorrichtung 15, insbesondere des Patiententischs 16. Insbesondere werden hierbei die entsprechenden Ausgabebereiche 39 der Positionseingabeelemente 38 der Positionseingabeeinheit 31 von der Recheneinheit 33 angesteuert mit einem Ausgabesignal, insbesondere mit einem Positionskontrollsignal, zur Ausgabe and den entsprechenden Ausgabebereichen 39. Das Ausgabesignal, insbesondere das Positionskontrollsignal, wird von der Recheneinheit 33 nach Erhalt des Positionseingangssignals und/oder nach Auslesen der Positionseingabeelemente 35 generiert.

Die Ausgabe eines Positionskontrollsignals mittels der Positionsausgabeeinheit 31 erfolgt bevorzugt an einer Position, die in Richtung einer Längserstreckung 37 des Patiententischs 16 mit der Position einer Positionseingabe mittels der Positionseingabeeinheit 30 übereinstimmt. Durch die Ausgabe eines Positionskontrollsignals an einer Position einer Positionseingabe kann der Benutzer, insbesondere das medizinische Bedienpersonal direkt kontrollieren, ob die Eingabe korrekt vom System umfasst ist und ob die Position, insbesondere die von der Positionseingabeeinheit 30 erfasste Untersuchungsposition 42, an der korrekten Position ist.

Die Ausgabe des Positionskontrollsignals kann dabei ein optisches Positionskontrollsignal mittels des optischen Positionsausgabeelements 38 und/oder ein haptisches Positionskontrollsignal mittels des haptischen Positionsausgabeelements 38 umfassen. Das optische Positionskontrollsignal kann dabei ein Aufleuchten eines Ausgabebereichs 39 des Positionsausgabeelements 38 umfassen. Das haptische Positionskontrollsignal kann ein Vibrieren eines Ausgabebereichs 39 des Positionsausgabeelements 38 umfassen.

Zudem kann es sein, dass zur Kontrolle einer Eingabe eines zweiten Untersuchungsbereichs 42 das Positionskontrollsignal ein zweifaches Aufleuchten eines Ausgabebereichs 39 des Positionsausgabeelements 38 und/oder ein zweifaches Vibrieren eines Ausgabebereichs 39 des Positionsausgabeelements 38 umfasst. Zur Kontrolle einer Eingabe eines dritten Untersuchungsbereichs 42 kann das Positionskontrollsignal ein zweifaches Aufleuchten eines Ausgabebereichs 39 des Positionsausgabeelements 38 und/oder ein zweifaches Vibrieren eines Ausgabebereichs 39 des Positionsausgabeelements 38 umfassen.

Zudem kann es sein, dass zur Kontrolle einer Eingabe eines Untersuchungsbereichs 42 das Positionskontrollsignal ein Aufleuchten und/oder Vibrieren der Bereichsstartposition 41 und der Bereichsendposition 42 umfasst. Zudem kann es auch vorgesehen sein, dass zur Kontrolle einer Eingabe eines Untersuchungsbereichs das Positionskontrollsignal ein Aufleuchten und/oder Vibrieren des gesamten mittels der Positionseingabeeinheit 30 erfassten Untersuchungsbereichs umfasst.

Zudem kann es auch sein, dass grundsätzlich die Positionseingabeeinheit 30, insbesondere die einzelnen Eingabebereiche 36 der Positionseingabeelemente 35, zur besseren Sichtbarkeit für einen Benutzer, insbesondere ein medizinisches Bedienpersonal, beleuchtet sind. Eine Ausgabe eines Positionskontrollsignal, insbesondere eines optischen Positionskontrollsignals, kann dabei farblich abgesetzt zur Ausleuchtung der Eingabebereiche 36 erfolgen. Für eine Ausleuchtung der Eingabebereiche 36 der Positionseingabeeinheit 30 können die Positionseingabeelemente 35 mit den Positionsausgabeelemente 38 einstückig und/oder einteilig ausgebildet sein. Zudem können auch bei einer getrennten und/oder separaten Ausbildung der Positionseingabeelemente 35 mit den Positionsausgabeelementen 38 die Positionseingabeelemente 35 jeweils zumindest ein Leuchtelement, wie beispielsweise eine LED-Element, umfassen zur Beleuchtung der Eingabebereiche 36 der Positionseingabeelemente 35.

Für eine Eingabe der zumindest einen Untersuchungsposition mittels der Positionseingabeeinheit 30, insbesondere der Positionseingabeelemente 35, ist der Patiententisch 16 in einer Home-Position außerhalb des Patientenaufnahmebereichs 13 positioniert.

In einem weiteren, optionalen Verfahrensschritt 102 erfolgt ein Einfahren des Patiententischs in den Patientenaufnahmebereich13. Das Einfahren des Patiententischs 16 wird von der Recheneinheit 33 gesteuert. Hierbei wird der Patiententisch 16 soweit in den Patientenaufnahmebereich 13 eingefahren, bis der Untersuchungsbereich 42 innerhalb des FOVs 43 angeordnet ist und/oder mit dem Isozentrum der Magnetresonanzvorrichtung 11 übereinstimmt. Sobald jedoch der Patiententisch 16 die Home-Position verlässt bzw. nicht mehr in dieser angeordnet und/oder positioniert ist, wird in diesem optionalen Verfahrensschritt 102 die Positionseingabeeinheit 30 deaktiviert. Die Deaktivierung der Positionseingabeeinheit 30 wird von der Recheneinheit 33 automatisch gesteuert. Zudem wird von der Recheneinheit 33 die Positionseingabeeinheit 30 automatisch aktiviert, sobald der Patiententisch 16 in der Home-Position ist.

Die Positionseingabeeinheit 30 ist zudem dazu ausgelegt und/oder ausgebildet, eine Eingabegeste des Benutzers in eine einfahrende Richtung des Patiententischs 16 zu erfassen. Mittels der erfassten Bewegungsgeste in die einfahrende Richtung kann vom Benutzer eine Einfahrbewegung des Patiententischs 16 in einen Patientenaufnahmebereich 13 gestartet werden. Nach Erfassen der Bewegungsgeste in die einfahrende Richtung des Patiententischs 16 wird die Einfahrbewegung des Patiententischs 16 gestartet und der Patiententisch verlässt seine Home-Position, um in den Patientenaufnahmebereich 13 zu fahren. Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen, welcher in den anhängenden Ansprüchen definiert ist.

## Patentansprüche

1. Verfahren zu einem Positionieren eines Patienten (14) für eine medizinische Bildgebungsuntersuchung, insbesondere eine Magnetresonanzuntersuchung, mittels einer Patientenlagerungsvorrichtung (15) mit einem bewegbaren Patiententisch (16), wobei der bewegbare Patiententisch zu einem Einfahren in einen Patientenaufnahmebereich (13) einer medizinischen Bildgebungsvorrichtung (10) ausgebildet ist, und einer Positionierungseinheit (29) mit einer Positionseingabeeinheit(30), die an einem seitlichen Randbereich des Patiententisch angeordnet ist, wobei die Positionierungseinheit eine Positionsausgabeeinheit (31) umfasst, die an dem seitlichen Randbereich des Patiententischs angeordnet ist, wobei das Verfahren die folgenden Verfahrensschritte umfasst:
- Erfassen einer Untersuchungsposition (42) mittels der Positionseingabeeinheit,
- Ausgabe eines Positionskontrollsignals mittels der Positionsausgabeeinheit,
**dadurch gekennzeichnet, dass** für die medizinische Bildgebungsuntersuchung n Untersuchungspositionen mittels der Positionseingabeeinheit eingebbar sind, wobei eine n-te Untersuchungsposition durch ein n-faches Betätigen der Positionseingabeeinheit festgelegt wird.

2. Verfahren nach Anspruch 1, **dadurch gekennzeichnet, dass** das Erfassen der Untersuchungsposition ein Betätigen eines drucksensitives Positionseingabeelements (35) der Positionseingabeeinheit umfasst, wobei die Positionseingabeeinheit zwei oder mehr drucksensitive Bereiche umfasst, wobei die einzelnen drucksensitiven Bereiche in Richtung einer Längserstreckung des drucksensitiven Positionseingabeelements nacheinander angeordnet sind.

3. Verfahren nach einem der vorhergehenden Ansprüche, **dadurch gekennzeichnet, dass** das Erfassen der Untersuchungsposition ein Betätigen eines Positionseingabeelements der Positionseingabeeinheit an einer Position umfasst, wobei die Position in Richtung einer Längserstreckung des Patiententischs mit dem zu untersuchenden Bereich des Patienten übereinstimmt.

4. Verfahren nach einem der vorhergehenden Ansprüche, **dadurch gekennzeichnet, dass** das Erfassen der Untersuchungsposition eine Eingabe eines Untersuchungsbereichs umfasst, wobei die Eingabe des Untersuchungsbereichs ein Festlegen einer Bereichsstartposition durch Kontaktierung des Benutzers mit einem Eingabebereich der Positionseingabeeinheit und einer kontinuierlichen Kontaktierung der Positionseingabeeinheit bis zu einem Erreichen einer Bereichsendposition umfasst.

5. Verfahren nach einem der vorhergehenden Ansprüche, **dadurch gekennzeichnet, dass** die Ausgabe des Positionskontrollsignals mittels der Positionsausgabeeinheit erfolgt, wobei die Positionsausgabeeinheit ein Positionsausgabeelement mit zwei oder mehr Ausgabebereichen umfasst, die in Richtung einer Längserstreckung des zumindest einen Positionsausgabeelements nacheinander angeordnet sind.

6. Verfahren nach einem der vorhergehenden Ansprüche, **dadurch gekennzeichnet, dass** die Ausgabe des Positionskontrollsignals mittels der Positionsausgabeeinheit an einer Position erfolgt, welche Position in Richtung einer Längserstreckung des Patiententischs mit einer Position einer Positionseingabe mittels der Positionseingabeeinheit übereinstimmt.

7. Verfahren nach einem der vorhergehenden Ansprüche, **dadurch gekennzeichnet, dass** die Ausgabe des Positionskontrollsignals eine Ausgabe eines optischen Positionskontrollsignals und/oder haptischen Positionskontrollsignals umfasst.

8. Verfahren nach einem der vorhergehenden Ansprüche, **dadurch gekennzeichnet, dass** der Patiententisch für eine Eingabe der zumindest einen Untersuchungsposition in einer Home-Position außerhalb des Patientenaufnahmebereichs der medizinischen Bildgebungsvorrichtung positioniert ist, wobei die Patienteneingabeeinheit deaktiviert wird, sobald der Patiententisch die Home-Position verlässt.

9. Verfahren nach einem der vorhergehenden Ansprüche, **dadurch gekennzeichnet, dass** eine Bewegungsgeste des Benutzers in eine einfahrende Richtung an der Positionseingabeeinheit erfasst wird, wobei die erfasste Bewegungsgeste in die einfahrende Richtung eine Einfahrbewegung des Patiententischs in einen Patientenaufnahmebereich startet.

## Claims

1. Method for positioning a patient (14) for a medical imaging examination, in particular a magnetic resonance examination, by means of a patient positioning apparatus (15) with a moveable patient couch (16), wherein the moveable patient couch is designed to be introduced into a patient receiving region (13) of a medical imaging apparatus (10), and with a positioning unit (29) with a position input unit (30) which is arranged on a lateral edge region of the patient couch, wherein the positioning unit comprises a position output unit (31) which is arranged on the lateral edge region of the patient couch, wherein the method comprises the following method steps:
- detect an examination position (42) by means of the position input unit,
- output a position control signal by means of the position output unit,
**characterised in that**, for the medical imaging examination, n examination positions can be input by means of the position input unit, wherein an nth examination position is specified by way of an n-fold actuation of the position input unit.

2. Method according to claim 1,
**characterised in that** the detection of the examination position comprises an actuation of a pressure-sensitive position input element (35) of the position input unit, wherein the position input unit comprises two or more pressure-sensitive regions, wherein the individual pressure-sensitive regions are arranged in succession in the direction of a longitudinal extension of the pressure-sensitive position input element.

3. Method according to one of the preceding claims, **characterised in that** the detection of the examination position comprises an actuation of a position input element at one position of the position input unit, wherein the position in the direction of a longitudinal extension of the patient couch corresponds with the region of the patient to be examined.

4. Method according to one of the preceding claims, **characterised in that** the detection of the examination position comprises an input of an examination region, wherein the input of the examination region comprises specification of a region start position by way of the user's contact with an input region of the position input unit and a continuous contact of the position input unit until a region end position is reached.

5. Method according to one of the preceding claims, **characterised in that** the output of the position control signal takes place by means of the position output unit, wherein the position output unit comprises a position output element with two or more output regions, which are arranged in succession in the direction of a longitudinal extension of the at least one position output element.

6. Method according to one of the preceding claims, **characterised in that** the output of the position control signal takes place at one position by means of the position output unit, which position, in the direction of a longitudinal extension of the patient couch, corresponds with a position of a position input by means of the position input unit.

7. Method according to one of the preceding claims, **characterised in that** the output of the position control signal comprises an output of an optical position control signal and/or a haptic position control signal.

8. Method according to one of the preceding claims, **characterised in that**, for an input of the at least one examination position, the patient couch is positioned in a home position externally to the patient receiving region of the medical imaging apparatus, wherein the patient input unit is deactivated as soon as the patient couch vacates the home position.

9. Method according to one of the preceding claims, **characterised in that** a user's motion gesture is detected at the position input unit in a direction of entry, wherein the detected motion gesture in the direction of entry starts an entry movement of the patient couch into a patient receiving region.

## Revendications

1. Procédé de mise en position d'un patient (14) pour un examen d'imagerie médicale, en particulier un examen par résonance magnétique, au moyen d'une installation (15) d'étendue du patient ayant une table (16) de patient mobile,
dans lequel la table de patient mobile est constituée pour une entrée dans une partie (13) d'enregistrement du patient d'une installation (10) d'imagerie médicale et une unité (29) de mise en position ayant une unité (30) d'entrée de position, qui est disposée sur une partie de bord latérale de la table de patient, dans lequel l'unité de mise en position comprend une unité (31) de sortie de position, qui est disposée sur la partie de bord latérale de la table de patient,
dans lequel le procédé comprend les stades de procédé suivants :
- détection d'une position (42) d'examen, au moyen de l'unité d'entrée de position,
- sortie d'un signal de commande de position, au moyen de l'unité de sortie de position,
**caractérisé en ce que**, pour l'examen d'imagerie médicale on peut entrer n positions d'examen au moyen de l'unité d'entrée de position, une énième position d'examen étant fixée par un actionnement n fois de l'unité d'entrée de position.

2. Procédé suivant la revendication 1,
**caractérisé en ce que** la détection de la position d'examen comprend un actionnement d'un élément (35) d'entrée de position sensible à la pression de l'unité d'entrée de position, dans lequel l'unité d'entrée de position comprend deux ou plusieurs parties sensibles à la pression, dans lequel les diverses parties sensibles à la pression sont disposées les unes après les autres dans la direction d'une étendue longitudinale de l'élément d'entrée de position sensible à la pression.

3. Procédé suivant l'une des revendications précédentes,
**caractérisé en ce que** la détection de la position d'examen comprend un actionnement d'un élément d'entrée de position de l'unité d'entrée de position à une position, dans lequel la position coïncide dans la direction d'une étendue longitudinale de la table du patient avec la partie à examiner du patient.

4. Procédé suivant l'une des revendications précédentes,
**caractérisé en ce que** la détection de la position d'examen comprend une entrée d'une partie à examiner, dans lequel l'entrée de la partie à examiner comprend une détermination d'une position de début de partie par mise en contact de l'utilisateur avec une partie d'entrée de l'unité d'entrée de position et une mise en contact continue de l'unité d'entrée et de position jusqu'à obtenir une position finale de la partie.

5. Procédé suivant l'une des revendications précédentes,
**caractérisé en ce que** la sortie du signal de commande de position s'effectue au moyen de l'unité de sortie de position, dans lequel l'unité de sortie de position comprend un élément de sortie de position ayant deux ou plusieurs parties de sortie, qui sont disposées les unes derrière les autres dans la direction d'étendue longitudinale du au moins un élément de sortie de position.

6. Procédé suivant l'une des revendications précédentes,
**caractérisé en ce que** la sortie du signal de commande de position s'effectue au moyen de l'unité de sortie de position en une position, laquelle position coïncide dans la direction d'une étendue longitudinale de la table du patient avec une position d'une entrée de position au moyen de l'unité d'entrée de position.

7. Procédé suivant l'une des revendications précédentes,
**caractérisé en ce que** la sortie du signal de commande de position comprend une sortie d'un signal de commande de position optique et/ou d'un signal de commande de position haptique.

8. Procédé suivant l'une des revendications précédentes,
**caractérisé en ce que** la table de patient est, pour une entrée de la au moins une position d'examen, mise en position dans une position de mise à disposition à l'extérieur de la partie d'enregistrement du patient de l'installation d'imagerie médicale, dans lequel on désactive l'unité d'entrée du patient dès que la table du patient quitte la position de mise à disposition.

9. Procédé suivant l'une des revendications précédentes,
**caractérisé en ce que** l'on détecte un geste de déplacement de l'utilisateur dans un sens d'entrée à l'unité d'entrée de position, dans lequel le geste de déplacement, détecté dans le sens d'entrée, fait débuter un déplacement d'entrée de la table du patient dans une partie d'enregistrement du patient.
